# EUROPÄISCHE PATENTSCHRIFT

(11) **EP 3 335 691 B1**
(45) Veröffentlichungstag und Bekanntmachung des Hinweises auf die Patenterteilung: **11.12.2019**
(21) Anmeldenummer: 17202737.7
(22) Anmeldetag: 08.10.2015
(51) Int. Cl.: A61K 8/35, A61K 8/41, A61K 8/49, A61K 8/73, A61Q 17/04, C11D 3/00, D06M 15/03

(54) **POLYSACCHARID-HALTIGE SONNENSCHUTZMITTEL MIT REDUZIERTER NEIGUNG ZUR TEXTILVERFLECKUNG**
POLYSACCHARIDE-CONTAINING SUNSCREEN COMPOSITION WITH A REDUCED LIKELIHOOD OF DISCOLOURING TEXTILES
PRÉPARATION ANTI-SOLAIRE CONTENANT DES POLYSACCHARIDES PRÉSENTANT UNE TENDANCE RÉDUITE À TÂCHER LES TISSUS

(30) Priorität: 22.10.2014 DE 102014015554
(43) Veröffentlichungstag der Anmeldung: 20.06.2018
(62) Teilanmeldung aus: 15787147.6
(73) Patentinhaber: Beiersdorf AG, 20253 Hamburg (DE)
(72) Erfinder: Weinert, Katrin, 22763 Hamburg (DE); Borchers, Kathrin, 21614 Buxtehude (DE); Bleckmann, Andreas, 22926 Ahrensburg (DE)

(56) Entgegenhaltungen:
- EP-A2- 2 647 366
- DE-A1-102010 008 320
- US-A1- 2013 309 185

## Beschreibung

Die vorliegende Erfindung betrifft die Verwendung von Polysacchariden in UV-Filter enthaltenden kosmetischen Zubereitungen, zur Reduzierung der durch die UV-Filter in der Zubereitung hervorgerufenen Verunreinigung und/oder Verfärbung von Textilien sowie zur leichteren Auswaschbarkeit der durch die UV-Filter in der Zubereitung hervorgerufenen Verunreinigung und/oder Verfärbung von Textilien, sowie entsprechende Verfahren zur leichteren Auswaschbarkeit der Verfleckungen.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurde daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

Kosmetische Zubereitungen wie Sonnenschutzzubereitungen, die auf die Haut aufgetragen werden, kommen regelmäßig (beabsichtigt oder unbeabsichtigt) mit Kleidungsstücken und Wäschestücken (z.B. Handtücher) in Kontakt, an denen sie (z.B. als "Abrieb" oder weil sie von den Faserstoffen "aufgesaugt" werden) zum Teil haften bleiben. Auf diese Weise entstehen, je nach Art der Inhaltsstoffe, insbesondere auf hellen Textilien, Flecken und Verfärbungen. Diese Verfärbungen werden vor allem durch nicht-wasserlösliche UVA- und Breitbandfilter hervorgerufen, insbesondere durch die UV-Filter 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin), 4-(tert.-Butyl)-4'-methoxydibenzoylmethan (INCI Butyl Methoxydibenzoylmethane), und (2-[-4-(Diethylamino)-2-hydroxybenzoyl] Benzoesäurehexylester (INCI: Diethylamino Hydroxybenzoyl Hexyl Benzoate). Die Verfleckungen sind durch Waschen mit herkömmlichen Waschmitteln kaum zu entfernen und verstärken sich während des Waschprozesses durch Wechselwirkungen mit Ionen des Waschwassers sogar noch. Das Problem tritt besonders stark bei Zubereitungen mit hohem Lichtschutzfaktor auf.

Der Einsatz von Polysacchariden zur Vermeidung der Textilverfleckung ist Gegenstand der Patentschriften DE102010008320 und EP2647366.

Es war daher die Aufgabe der vorliegenden Erfindung, die Nachteile des Standes der Technik zu beseitigen und eine kosmetische Zubereitung (insbesondere ein Sonnenschutzmittel) enthaltend nicht-wasserlösliche UV-A Filter wie 4-(tert.-Butyl)-4'-methoxydibenzoylmethan (INCI Butyl Methoxydibenzoylmethane) und (2-[-4-(Diethylamino)-2-hydroxybenzoyl] Benzoesäurehexylester (INCI: Diethylamino Hydroxybenzoyl Hexyl Benzoate) und Breitbandfilter wie Bis-Ethylhexyloxyphenol Methoxyphenyl Triazine zu entwickeln, welche sich leichter aus den mit der Zubereitung kontaminierten Textilen herauswaschen lassen. Überraschend gelöst wird die Aufgabe durch die Verwendung von Carrageenan in 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin) enthaltenden kosmetischen Zubereitungen, zur Reduzierung der durch die UV-Filter in der Zubereitung hervorgerufenen Verunreinigung und/oder Verfärbung von Textilien,
sowie
durch die Verwendung von Carrageenan in 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin) enthaltenden kosmetischen Zubereitungen, zur leichteren Auswaschbarkeit der durch die UV-Filter in der Zubereitung hervorgerufenen Verunreinigung und/oder Verfärbung von Textilien.

Überraschend gelöst wird die Aufgabe ferner durch ein Verfahren zur Erleichterung der Auswaschbarkeit von 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin) enthaltenden kosmetischen Zubereitungen aus Textilien, dadurch gekennzeichnet, dass dem Kosmetikum Carrageenan zugesetzt wird sowie durch ein Verfahren zur Reduzierung der durch 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin) enthaltenden kosmetischen Zubereitungen hervorgerufenen Verfärbung, dadurch gekennzeichnet, dass dem Kosmetikum Carrageenan zugesetzt wird.

Zwar sind dem Fachmann durchaus Sonnenschutzmittel mit Polysachariden bekannt, doch wurden diese bislang zur Verbesserung der sensorischen Eigenschaften (Puderrohstoff) bei der Anwendung der Produkte *auf der Haut* oder zur Stabilisierung/ Verdickung der Formulierungen eingesetzt. Dass Polysaccharide jedoch einen Einfluss auf die unerwünschte Textilverfleckung haben und insbesondere zu einer leichteren Auswaschbarkeit des Sonnenschutzmittels und seiner UV-Filter aus den Textilen heraus, führen war bisher unbekannt und auch nicht zu erwarten gewesen.

Im Rahmen der vorliegenden Offenbarung beziehen sich die Formulierungen "erfindungsgemäß", "erfindungsgemäße Zubereitung" etc. immer auf die erfindungsgemäßen Verfahren und Verwendungen, d.h. auf Zubereitungen, in denen die erfindungsgemäßen Verwendungen verwirklicht werden sowie Zubereitungen, mit denen das erfindungsgemäße Verfahren verwirklicht wird.

Erfindungsgemäße Ausführungsformen der vorliegenden Erfindung sind dadurch gekennzeichnet, dass die Zubereitung 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin) als verunreinigende und/oder verfärbende UV-Filter enthält.

Es ist in jedem Falle erfindungsgemäß vorteilhaft, wenn die Zubereitung Polysaccharide in einer Gesamtmenge von 0,01 bis 2 Gewichts-%, bezogen auf das Gesamtgewicht der Zubereitung, enthält.

Für die erfindungsgemäß vorteilhaften Polysaccharide gelten für die einzelnen Stoffe die folgenden Einsatzkonzentrationen als erfindungsgemäß bevorzugt:
Welan Gum wird erfindungsgemäß bevorzugt in einer Konzentration von 0,01 bis 1,5 Gewichts-%, bezogen auf das Gesamtgewicht der Zusammensetzung eingesetzt. Als Welan Gum kann beispielsweise der Rohstoff Collstab W-100 der Firma Colltec vorteilhaft eingesetzt werden.

Sclerotium Gum wird erfindungsgemäß bevorzugt in einer Konzentration von 0,01 bis 1,5 Gewichts-%, bezogen auf das Gesamtgewicht der Zusammensetzung eingesetzt. Als Sclerotium Gum kann beispielsweise der Rohstoff Actigum CS 11 der Firma Cargill vorteilhaft eingesetzt werden.

Cellulose Gum wird erfindungsgemäß bevorzugt in einer Konzentration von 0,01 bis 1,5 Gewichts-%, bezogen auf das Gesamtgewicht der Zusammensetzung eingesetzt. Als Cellulose Gum kann beispielsweise der Rohstoff Blanose Cellulose Gum der Firma Ashland vorteilhaft eingesetzt werden.

Sodium Alginate wird erfindungsgemäß bevorzugt in einer Konzentration von 0,01 bis 1,5 Gewichts-%, bezogen auf das Gesamtgewicht der Zusammensetzung eingesetzt. Als Sodium Alginate kann beispielsweise der Rohstoff Alginic Acid Sodium Salt der Firma Sigma Aldrich vorteilhaft eingesetzt werden.

Carboxymethylcellulose wird erfindungsgemäß bevorzugt in einer Konzentration von 0,01 bis 2 Gewichts-%, bezogen auf das Gesamtgewicht der Zusammensetzung eingesetzt. Als Carboxymethylcellulose kann beispielsweise der Rohstoff Aqualon CMC der Firma Ashland vorteilhaft eingesetzt werden.

Erfindungsgemäß besonders bevorzugt wird für die erfindungsgemäße Verwendung bzw. das erfindungsgemäße Verfahren Carrageenan, Cellulose Gum und/oder Alginate (insbesondere Natriumalginat) eingesetzt, wenn die Auswaschbarkeit von 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin) erleichtert werden soll (bzw. durch diesen UV-Filter hervorgerufene Textilverfleckung reduziert werden soll).

Erfindungsgemäß vorteilhafte Ausführungsformen der vorliegenden Erfindung sind dadurch gekennzeichnet, dass die Zubereitung eine oder mehrere der Säure- bzw. Salzverbindungen aus der Gruppe
- 1-Hydroxyethan-(1,1-diphosphonsäure)/ HEDP
- Aminotrimethylenphosphonsäure/ ATMP
- Diethylentriaminpenta(methylenphosphonsäure)/ DTPMP
- Ethylendiamintetra(methylenphosphonsäure/ EDTMP
- Phosphonobutan-tricarbonsäure/ PBTC
- Iminodisuccinat
- Natriumpolyphosphat
- Tetranatriumpyrophosphat
- Hydroxamsäure
- Polygalacturonsäure
- Bernsteinsäure
- Ameisensäure
- Äpfelsäure
- 1-Hydroxyethan-(1,1-diphosphonsäure)/ HEDP
- Aminotrimethylenphosphonsäure/ ATMP
- Diethylentriaminpenta(methylenphosphonsäure)/ DTPMP
- Ethylendiamintetra(methylenphosphonsäure/ EDTMP
- Phosphonobutan-tricarbonsäure/ PBTC
- Iminodisuccinat
- Natriumpolyphosphat
- Tetranatriumpyrophosphat
- Hydroxamsäure
- Polygalacturonsäure
- Bernsteinsäure
- Ameisensäure
- Äpfelsäure
- Ethylendiamintetraessigsäure (EDTA)
   und/oder deren Alkalisalze und/ oder deren Amin-N-Oxide enthält.

In einem solchen Fall ist es erfindungsgemäß bevorzugt, wenn die Zubereitung eine oder mehrere dieser Säure- bzw. Salzverbindungen in einer Gesamtmenge von 0,1 bis 3 Gewichts-%, bezogen auf das Gesamtgewicht der Zubereitung, enthält.

Grundsätzlich ist es bei der erfindungsgemäßen Verwendung und dem erfindungsgemäßen Verfahren bevorzugt, wenn die Zubereitung 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin) in einer Menge von 0,01 bis 10 Gewichts-%, bezogen auf das Gesamtgewicht der Zubereitung, enthält.

Außerdem ist es grundsätzlich bei der erfindungsgemäßen Verwendung und dem erfindungsgemäßen Verfahren bevorzugt, wenn die Zubereitung 4-(tert.-Butyl)-4'-methoxydibenzoylmethan (INCI Butyl Methoxydibenzoylmethane) in einer Menge von 0,01 bis 10 Gewichts-%, bezogen auf das Gesamtgewicht der Zubereitung, enthält.

Nicht zuletzt ist es grundsätzlich bei der erfindungsgemäßen Verwendung und dem erfindungsgemäßen Verfahren bevorzugt, wenn die Zubereitung (2-[-4-(Diethylamino)-2-hydroxybenzoyl] Benzoesäurehexylester (INCI: Diethylamino Hydroxybenzoyl Hexyl Benzoate) in einer Menge von 0,01 bis 10 Gewichts-%, bezogen auf das Gesamtgewicht der Zubereitung, enthält.

Die Zubereitungen, in denen die Erfindung verwirklicht wird, können darüber hinaus erfindungsgemäß vorteilhaft weitere UV-Filter enthalten. Diese werden vorteilhaft gewählt aus der Gruppe der Verbindungen Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 2-Ethylhexyl-2-cyano-3,3-diphenylacrylat; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoesäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Titandioxid; Zinkoxid.

Es ist dabei erfindungsgemäß besonders bevorzugt, wenn die Zubereitung frei ist von 3-(4-Methylbenzyliden)-campher und 2-Hydroxy-4-methoxybenzophenon (Oxybenzon).

Erfindungsgemäß vorteilhafte Ausführungsformen können dadurch erhalten werden, dass die Zubereitung Ethylhexylglycerin, Polyglyceryl-2 Caprate, Propylenglycol, Butylenglycol, 2-Methylpropan-1,3-diol, 1,2-Pentandiol, 1,2-Hexandiol, 1,2-Octandiol und/oder 1,2-Decandiol enthält.

Es ist erfindungsgemäß vorteilhaft, wenn die erfindungsgemäße Zubereitung Phenoxyethanol und/oder Methylparaben enthält.

Es ist dabei erfindungsgemäß bevorzugt, wenn die Zubereitung frei ist von Propyl- und Butylparaben, 3-Iod-2-propinylbutylcarbamat,

Erfindungsgemäß vorteilhafte Ausführungsformen sind auch dadurch gekennzeichnet, dass die Zubereitung einen oder mehrere Wirkstoffe gewählt aus der Gruppe der Verbindungen Magnolienextrakt, Gylcyrrhetinsäure, Harnstoff, Arctiin, alpha-Liponsäure, Folsäure, Phytoen, D-Biotin, Coenzym Q10, Hyaluronsäure, Glycyrrhetinsäure, alpha-Glucosylrutin, Carnitin, Carnosin, Coffein, natürliche und/oder synthetische Isoflavonoide, Glycerylglucose, Kreatin, Kreatinin, Taurin, Tocopherol, Tocopherolacetat, β-Alanin, Panthenol und/oder Licochalcon A, enthält.

Die erfindungsgemäße Zubereitung kann ferner vorteilshaft Glycerin und/oder Ethanol enthalten. In einem solchen Falle ist eine Glycerin-Konzentration von 0,01 bis 10 Gewichts-%, bezogen auf das Gesamtgewicht der Zubereitung, erfindungsgemäß vorteilhaft. Für Ethanol liegt der erfindungsgemäß vorteilhafte Einsatzbereich zwischen 0,01 und 10 Gewichts-%, bezogen auf das Gesamtgewicht der Zubereitung.

Die Ölphase der erfindungsgemäßen Zubereitung kann darüber hinaus noch Öl-, Fett- und Wachskomponenten enthalten, beispielsweise polaren Öle aus der Gruppe der Lecithine oder Verbindungen wie z. B. Cocoglycerid, Capryl/Caprinsäure Triglycerid, Olivenöl, Sonnenblumenöl, Jojobaöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr. Auch Verbindungen wie Phenethylbenzoat, 2-Phenylethylbenzoat, Isopropyl Lauroyl Sarkosinat, Phenyl Trimethicon, Cyclomethicon, Dibutyladipat, Octylpalmitat, Octylcocoat, Octylisostearat, Octyldodeceylmyristat, Octyldodekanol, Cetearylisononanoat, Isopropylmyristat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat können eingesetzt werden.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* bei der Fa. Cognis erhältliche.

Es ist ferner vorteilhaft, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid gewählt werden. Es ist insbesondere vorteilhaft, wenn die Ölphase der erfindungsgemäßen Formulierungen einen Gehalt an C₁₂₋₁₅-Alkylbenzoat aufweist.

Vorteilhafte Ölkomponenten sind ferner z. B. Isopropylpalmitat, Myristylmyristat, Butyloctylsalicylat (beispielsweise das unter der Handelsbezeichnung *Hallbrite BHB* bei der Fa. CP Hall erhältliche), Tridecylsalicylat (welches unter der Handelsbezeichnung Cosmacol ESI bei der Fa. Sasol erhältlich ist), C12-C15 Alkylsalicylat (unter der Handelsbezeichnung Dermol NS bei der Fa. Alzo erhältlich), Hexadecylbenzoat und Butyloctylbenzoat und Gemische davon (*Hallstar AB*).

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene, C13-16 Isoparaffin und Isohexadecan. Unter den Polyolefinen sind Polydecene die bevorzugten Substanzen.

Erfindungsgemäß vorteilhafte Ausführungsformen der vorliegenden Erfindung sind auch dadurch gekennzeichnet, dass die Emulsion Dimethicone und/oder Cyclomethicon enthält.

Erfindungsgemäß besonders bevorzugt ist es, wenn die Ölphase der Zubereitung Di-n-Octylcarbonat (INCI Dicaprylyl Carbonate), Di-Isopropyladipat und/oder Di-n-Butyladipat (INCI Dibutyl Adipate) enthält.

Als polymeren Filmbildner zur Erhöhung der Wasserfestigkeit kann die erfindungsgemäße Zubereitung erfindungsgemäß vorteilhaft Vinylpyrrolidon/Hexadecen Copolymer enthalten. Darüber hinaus ist der Zusatz Tapiokastärke erfindungsgemäß vorteilhaft.

Darüber hinaus kann die erfindungsgemäße Zubereitung die für die Kosmetik üblichen Inhaltsstoffe in den entsprechenden Einsatzkonzentrationen enthalten.

Die erfindungsgemäßen Zubereitungen liegen vorteilhaft als Emulsion, Hydrodispersion oder alkoholische Lösung vor. Erfindungsgemäße bevorzugt ist dabei die Emulsion, insbesondere die öl-in-Wasser-Emulsion (O/W-Emulsion).

Die erfindungsgemäße Zubereitung kann insbesondere vorteilhaft als Tagespflegeprodukt oder Sonnenschutzmittel eingesetzt werden.

### Vergleichsversuch

Mit dem folgenden Versuch konnte der erfindungsgemäße Effekt beispielhaft belegt werden:
Es wurde 1% des erfindungsgemäßen Carrageenans zu einer Bis-Ethylhexyloxyphenol Methoxyphenyl Triazin enthaltenden Formulierung zugesetzt und die verfleckungsreduzierende Wirkung (Reduktion b*) im Vergleich zu einer Formulierung ohne erfindungsgemäße Polysaccharide mittels beschriebener Methode bestimmt. Als Beleg der verbesserten Auswaschbarkeit und verminderten Fleckenbildung der erfindungsgemäßen Zubereitungen wurden in vitro Untersuchungen durchgeführt, deren Ergebnisse in den Tabellen 1 und 2 dargestellt sind.

Es wurden verschiedene Sonnenschutzemulsionen hinsichtlich der Bildung von gelben

Flecke über einen in vitro Auftragungs-/ Waschzyklus untersucht. Es wurden dabei weiße vorgewaschene Baumwollmonitore (100% Baumwolle) verwendet. Dazu wurden je 25 mg der Test-Formulierung gleichmäßig auf PMMA Schönberg Platten (5,0 x 5,0 cm) verteilt und direkt mittels Andruck auf das Testtextil übertragen. Im Anschluss wurden die verfleckten Baumwollproben für 12h unter Laborbedingungen an der Luft getrocknet.

Nach der Trocknung erfolgte eine farbmetrische Charakterisierung der entstandenen Initial-Verfleckung durch Messung des Gelbgrades mit dem Farbmessgerät spectro-color (Dr. Lange); Farbmess-Software: spectral-QC, Version Messgeometrie: d/8°, Glanzkomponente ausgeschlossen, Lichtart: D65 (entsprechend mittlerem Tageslicht), Kalibrierstandard: LZM 268, Messöffnung: 10mm, Probenhintergrund: Unterlagepapier ohne optischen Aufheller, Prüfklima: 21 °C (±1°C), 41% (±4%) rel. Luftfeuchte.

Zur Auswertung wurde die Veränderung des b-Wertes aus dem CIE-Lab Farbmesssystem herangezogen. Die B-Achse charakterisiert im CIE-Lab System den Farbeindruck Gelb/ Blau, wobei positive b-Werte für eine Zunahme des Gelbanteils stehen. Je höher der b-Wert desto größer ist der Gelbeindruck.

Nach dem Messvorgang erfolgte eine separate Wäsche der Testlappen im Färbe- und Waschechtheitsgerät Linitest Plus (Atlas) (60°C, 1h, 20rpm, Ariel Compact

Pulverwaschmittel, zehn Metallkugeln als Beiladung) und im Anschluss ein Spülvorgang (20°C, 15min, Leitungswasser).

Nach Trocknung für 12h unter Laborbedingungen erfolgte erneut eine farbmetrische Charakterisierung der entstandenen Verfleckung durch Messung der Farbwerte wie bereits beschrieben mit dem Farbmessgerät spectro-color (Dr. Lange).

Die CIE-Lab System oder L*a*b*-Farbraum ist ein dreidimensionaler Messraum, in dem alle wahrnehmbaren Farben enthalten sind. Der Farbraum ist auf Grundlage der Gegenfarbentheorie konstruiert. Eine der wichtigsten Eigenschaften des L*a*b*-Farbmodells ist seine Geräteunabhängigkeit, das heißt, die Farben werden unabhängig von der Art ihrer Erzeugung und Wiedergabetechnik definiert.

Die entsprechende EU-Richtlinie ist DIN EN ISO 11664-4 "Farbmetrik - Teil 4: CIE 1976 L*a*b* Farbenraum". Die Koordinaten der CIELAB-Ebene werden gebildet aus dem Rot/ Grün-Wert a und dem Gelb/ Blau-Wert b. Die Helligkeitsachse L steht senkrecht auf dieser Ebene. Nach DIN 6174 sind L, a und b mit * zu schreiben, um sich gegen andere, z.B. das "Hunter-Lab"-System abzugrenzen.

**Tabelle 1: getestete Zubereitungen und deren Gelbwert-Reduktion von Flecken; b*-Wert [%]**

| **INCI** | **Bsp.1** | **Bsp.2*** |
|---|---|---|
| Carrageenan | 1,0 | |
| Caprylic/Capric Triglyceride | 4,0 | 4,0 |
| Glyceryl Stearate | 1,0 | 1,0 |
| Hydrogenated Coco-Glycerides | 1,0 | 1,0 |
| Sodium Stearoyl Glutamate | 0,3 | 0,3 |
| Tapioca Stärke | 1,0 | 1,0 |
| Silica Dimethyl Silylat | 0,3 | 0,3 |
| Parfum | 0,5 | 0,5 |
| Glycerin | 0,9 | 0,9 |
| Phenoxyethanol | 0,6 | 0,6 |
| Methylparaben | 0,3 | 0,3 |
| Acrylates/C10-30 Alkyl Acrylate Crosspolymer | 0,2 | 0,2 |
| Xanthan Gummi | 0,4 | 0,4 |
| Alcohol Denat. | 8,0 | 8,0 |
| Trisnatrium EDTA | 0,2 | 0,2 |
| Octocrylen | 9,0 | 9,0 |
| Bis-Ethylhexyloxyphenol Methoxyphenyl Triazine | 3,0 | 3,0 |
| Wasser | ad. 100 | ad. 100 |
| | | |
| Reduktion b* [%] | -57 | 0 |

| | | |
|---|---|---|
| * Vergleichsformulierung ohne erfindungsgemäße Polysaccharide | | |

### Beispiel

Das nachfolgende Beispiel soll die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

**Tabelle 2: Übersicht Beispielrezeptur**

| **INCI** | |
|---|---|
| | **1** |
| Carrageenan | 1,0 |
| Trisodium EDTA | 0,20 |
| Butyl methoxydibenzoylmethane | 4,50 |
| Bis-Ethylhexyloxyphenol Methoxyphenyl Triazine | 3,50 |
| Phenylbenzimidazole Sulfonic Acid | 1,00 |
| Titanium Dioxide | 3,00 |
| Trimethoxycaprylylsilane | 0,20 |
| Octocrylene | 9,00 |
| Homosalate | 9,50 |
| Xanthan Gum | 0,40 |
| Acrylates/C10-30 Alkyl Acrylate Crosspolymer | 0,30 |
| Alcohol Denat. | 5,00 |
| Methylparaben | 0,30 |
| Phenoxyethanol | 0,60 |
| Citric Acid | 0,30 |
| Sodium Citrate | 0,10 |
| Sodium Hydroxide | 0,20 |
| Glycerin | 3,00 |
| Parfum | 0,40 |
| VP/Hexadecene Copolymer | 0,50 |
| Glyceryl Stearate Citrate | 2,00 |
| Hydrogenated Coco-Glycerides | 1,00 |
| C12-15 Alkyl Benzoate | 5,00 |
| Myristyl Myristate | 1,00 |
| Stearyl Alcohol | 0,50 |
| Isopropyl Stearate | 2,00 |
| Butylene Glycol Dicaprylate/Dicaprate | 5,00 |
| Aqua | ad 100 |

## Patentansprüche

1. Verwendung von Carrageenan in 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin) enthaltenden kosmetischen Zubereitungen, zur Reduzierung der durch die UV-Filter in der Zubereitung hervorgerufenen Verunreinigung und/oder Verfärbung von Textilien.

2. Verwendung von Carrageenan in 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin) enthaltenden kosmetischen Zubereitungen, zur leichteren Auswaschbarkeit der durch die UV-Filter in der Zubereitung hervorgerufenen Verunreinigung und/oder Verfärbung von Textilien.

3. Verfahren zur Erleichterung der Auswaschbarkeit von 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin) enthaltenden kosmetischen Zubereitungen aus Textilien, **dadurch gekennzeichnet, dass** dem Kosmetikum Carrageenan zugesetzt wird.

4. Verfahren zur Reduzierung der durch 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin) enthaltenden kosmetischen Zubereitungen hervorgerufenen Verfärbung von Textilien, **dadurch gekennzeichnet, dass** dem Kosmetikum Carrageenan zugesetzt wird.

5. Verwendung oder Verfahren nach einem der vorhergehenden Ansprüche, **dadurch gekennzeichnet, dass** die Zubereitung Polysaccharide in einer Gesamtmenge von 0,01 bis 2 Gewichts-%, bezogen auf das Gesamtgewicht der Zubereitung, enthält.

6. Verwendung oder Verfahren nach einem der vorhergehenden Ansprüche, **dadurch gekennzeichnet, dass** die Zubereitung eine oder mehrere Säure- bzw. Salzverbindungen aus der Gruppe
- 1-Hydroxyethan-(1,1-diphosphonsäure)/ HEDP
- Aminotrimethylenphosphonsäure/ ATMP
- Diethylentriaminpenta(methylenphosphonsäure)/ DTPMP
- Ethylendiamintetra(methylenphosphonsäure/ EDTMP
- Phosphonobutan-tricarbonsäure/ PBTC
- Iminodisuccinat
- Natriumpolyphosphat
- Tetranatriumpyrophosphat
- Hydroxamsäure
- Polygalacturonsäure
- Bernsteinsäure
- Ameisensäure
- Äpfelsäure
- 1-Hydroxyethan-(1,1-diphosphonsäure)/ HEDP
- Aminotrimethylenphosphonsäure/ ATMP
- Diethylentriaminpenta(methylenphosphonsäure)/ DTPMP
- Ethylendiamintetra(methylenphosphonsäure/ EDTMP
- Phosphonobutan-tricarbonsäure/ PBTC
- Iminodisuccinat
- Natriumpolyphosphat
- Tetranatriumpyrophosphat
- Hydroxamsäure
- Polygalacturonsäure
- Bernsteinsäure
- Ameisensäure
- Äpfelsäure
- Ethylendiamintetraessigsäure (EDTA)
und/oder deren Alkalisalze und/ oder deren Amin-N-Oxide enthält.

7. Verwendung oder Verfahren nach Anspruch 6, **dadurch gekennzeichnet, dass** die Zubereitung eine oder mehrere dieser Säure- bzw. Salzverbindungen in einer Gesamtmenge von 0,1 bis 3 Gewichts-%, bezogen auf das Gesamtgewicht der Zubereitung, enthält.

8. Verwendung oder Verfahren nach einem der vorhergehenden Ansprüche, **dadurch gekennzeichnet, dass** die Zubereitung ein oder mehrere Siloxanelastomere enthält.

9. Verwendung oder Verfahren nach Anspruch 8, **dadurch gekennzeichnet, dass** die Zubereitung eine oder mehrere Siloxanelastomere in einer Gesamtmenge von 0,01 bis 10 Gewichts-%, bezogen auf das Gesamtgewicht der Zubereitung, enthält.

10. Verwendung oder Verfahren nach einem der vorhergehenden Ansprüche, **dadurch gekennzeichnet, dass** die Zubereitung 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin) in einer Menge von 0,01 bis 10 Gewichts-%, bezogen auf das Gesamtgewicht der Zubereitung, enthält.

11. Verwendung oder Verfahren nach einem der vorhergehenden Ansprüche, **dadurch gekennzeichnet, dass** die Zubereitung 4-(tert.-Butyl)-4'-methoxydibenzoylmethan (INCI Butyl Methoxydibenzoylmethane) in einer Menge von 0,01 bis 10 Gewichts-%, bezogen auf das Gesamtgewicht der Zubereitung, enthält.

12. Verwendung oder Verfahren nach einem der vorhergehenden Ansprüche, **dadurch gekennzeichnet, dass** die Zubereitung (2-[-4-(Diethylamino)-2-hydroxybenzoyl] Benzoesäurehexylester (INCI: Diethylamino Hydroxybenzoyl Hexyl Benzoate) in einer Menge von 0,01 bis 10 Gewichts-%, bezogen auf das Gesamtgewicht der Zubereitung, enthält.

## Claims

1. Use of carrageenan in cosmetic preparations comprising 2,4-bis-{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine) for reducing soiling and/or discolouration of textiles caused by UV filters in the preparation.

2. Use of carrageenan in cosmetic preparations comprising 2,4-bis-{[4-(2-ethylhexyloxy)-2-hydroxy]phenyl}-6-(4-methoxyphenyl)-1,3,5- triazine (INCI: Bis-Ethylhexyloxyphenol Methoxyphenyl Triazine) for facilitating the ability to wash out soiling and/or discolouration from textiles caused by UV filters in the preparation.

3. Method for facilitating the ability to wash out cosmetic preparations comprising 2,4-bis-{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine)from textiles, **characterized in that** carrageenan is added to the cosmetic.

4. Method for reducing the discolouration of textiles caused by cosmetic preparations comprising 2,4-bis-{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine), **characterized in that** carrageenan is added to the cosmetic.

5. Use or method according to any of the preceding claims, **characterized in that** the preparation comprises polysaccharides in an amount of 0.01 to 2% by weight, based on the total weight of the preparation.

6. Use or method according to any of the preceding claims, **characterized in that** the preparation comprises one or more acid or salt compounds from the group comprising
- 1-hydroxyethane-(1,1-diphosphonic acid)/HEDP
- aminotrimethylene phosphonic acid/ATMP
- diethylenetriamine penta(methylenephosphonic acid)/DTPMP
- ethylenediamine tetra(methylenephosphonic acid)/EDTMP
- phosphonobutanetricarboxylic acid/PBTC
- iminodisuccinate
- sodium polyphosphate
- tetrasodium pyrophosphate
- hydroxamic acid
- polygalacturonic acid
- succinic acid
- formic acid
- malic acid
- 1-hydroxyethane-(1,1-diphosponic acid)/HEDP
- aminotrimethylene phosphonic acid/ATMP
- diethylenetriamine penta(methylenephosphonic acid)/DTPMP
- ethylenediamine tetra(methylenephosphonic acid)/EDTMP
- phosphonobutanetricarboxylic acid/PBTC
- iminodisuccinate
- sodium polyphosphate
- tetrasodium pyrophosphate
- hydroxamic acid
- polygalacturonic acid
- succinic acid
- formic acid
- malic acid
- ethylenediaminetetraacetic acid (EDTA)
and/or alkali metal salts thereof and/or amine N-oxides thereof.

7. Use or method according to Claim 6, **characterized in that** the preparation comprises one or more of these acid or salt compounds in a total amount of 0.1 to 3% by weight, based on the total weight of the compound.

8. Use or method according to any of the preceding claims, **characterized in that** the preparation comprises one or more siloxane elastomers.

9. Use or method according to Claim 8, **characterized in that** the preparation comprises one or more siloxane elastomers in a total amount of 0.01 to 10% by weight, based on the total amount of the preparation.

10. Use or method according to any of the preceding claims, **characterized in that** the preparation comprises 2,4-bis-{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine) in an amount of 0.01 to 10% by weight, based on the total weight of the preparation.

11. Use or method according to any of the preceding claims, **characterized in that** the preparation comprises 4-(tert-butyl)-4'-methoxydibenzoylmethane (INCI Butyl Methoxydibenzoylmethane) in an amount of 0.01 to 10% by weight, based on the total amount of the preparation.

12. Use or method according to any of the preceding claims, **characterized in that** the preparation comprises hexyl 2-[4-(diethylamino)-2-hydroxybenzoyl]benzoate (INCI: Diethylamino Hydroxybenzoyl Hexyl Benzoate) in an amount of 0.01 to 10% by weight, based on the total weight of the preparation.

## Revendications

1. Utilisation de carraghénane dans des préparations cosmétiques contenant de la 2,4-bis-{[4-(2-éthyl-hexyloxy)-2-hydroxy]-phényl}-6-(4-méthoxyphényl)-1,3,5-triazine (INCI : Bis-Ethylhexyloxyphenol Methoxyphenyl Triazin), pour réduire la contamination et/ou la coloration de textiles causées par les filtres UV dans la préparation.

2. Utilisation de carraghénane dans des préparations cosmétiques contenant de la 2,4-bis-{[4-(2-éthyl-hexyloxy)-2-hydroxy]-phényl}-6-(4-méthoxyphényl)-1,3,5-triazine (INCI : Bis-Ethylhexyloxyphenol Methoxyphenyl Triazin), pour faciliter la rinçabilité de la contamination et/ou de la coloration de textiles causées par les filtres UV dans la préparation.

3. Procédé visant à faciliter la rinçabilité de préparations cosmétiques contenant de la 2,4-bis-{[4-(2-éthyl-hexyloxy)-2-hydroxy]-phényl}-6-(4-méthoxyphényl)-1,3,5-triazine (INCI : Bis-Ethylhexyloxyphenol Methoxyphenyl Triazin) à partir de textiles, **caractérisé en ce que** de la carraghénane est ajoutée au cosmétique.

4. Procédé visant à réduire la coloration de textiles causée par des préparations cosmétiques contenant de la 2,4-bis-{[4-(2-éthyl-hexyloxy)-2-hydroxy]-phényl}-6-(4-méthoxyphényl)-1,3,5-triazine (INCI : Bis-Ethylhexyloxyphenol Methoxyphenyl Triazin), **caractérisé en ce que** de la carraghénane est ajoutée au cosmétique.

5. Utilisation ou procédé selon l'une quelconque des revendications précédentes, caractérisé ou **caractérisée en ce que** la préparation contient des polysaccharides en une quantité totale de 0,01 à 2 % en poids, par rapport au poids total de la préparation.

6. Utilisation ou procédé selon l'une quelconque des revendications précédentes, caractérisé ou **caractérisée en ce que** la préparation contient un ou plusieurs composés acides ou salins du groupe constitué par :
- l'acide 1-hydroxyéthane-(1,1-diphosphonique)/HEDP,
- l'acide aminotriméthylène-phosphonique/ ATMP,
- l'acide diéthylène-triamine-penta(méthylène-phosphonique)/ DTPMP,
- l'acide éthylène-diamine-tétra(méthylène-phosphonique)/ EDTMP,
- l'acide phosphonobutane-tricarboxylique/ PBTC,
- l'iminodisuccinate,
- le polyphosphate de sodium,
- le pyrophosphate de tétrasodium,
- l'acide hydroxamique,
- l'acide polygalacturonique,
- l'acide succinique,
- l'acide formique,
- l'acide malique,
- l'acide 1-hydroxyéthane-(1,1-diphosphonique)/ HEDP,
- l'acide aminotriméthylène-phosphonique/ ATMP,
- l'acide diéthylène-triamine-penta(méthylène-phosphonique)/ DTPMP,
- l'acide éthylène-diamine-tétra(méthylène-phosphonique)/ EDTMP,
- l'acide phosphonobutane-tricarboxylique (PBTC),
- l'iminodisuccinate,
- le polyphosphate de sodium,
- le pyrophosphate de tétrasodium,
- l'acide hydroxamique,
- l'acide polygalacturonique,
- l'acide succinique,
- l'acide formique,
- l'acide malique,
- l'acide éthylène-diamine-tétraacétique (EDTA),
et/ou leurs sels alcalins et/ou leurs amine-N-oxydes.

7. Utilisation ou procédé selon la revendication 6, caractérisé ou **caractérisée en ce que** la préparation contient un ou plusieurs de ces composés acides ou salins en une quantité totale de 0,1 à 3 % en poids, par rapport au poids total de la préparation.

8. Utilisation ou procédé selon l'une quelconque des revendications précédentes, caractérisé ou **caractérisée en ce que** la préparation contient un ou plusieurs élastomères de siloxane.

9. Utilisation ou procédé selon la revendication 8, caractérisé ou **caractérisée en ce que** la préparation contient un ou plusieurs élastomères de siloxane en une quantité totale de 0,01 à 10 % en poids, par rapport au poids total de la préparation.

10. Utilisation ou procédé selon l'une quelconque des revendications précédentes, caractérisé ou **caractérisée en ce que** la préparation contient de la 2,4-bis-{[4-(2-éthyl-hexyloxy)-2-hydroxy]-phényl}-6-(4-méthoxyphényl)-1,3,5-triazine (INCI : Bis-Ethylhexyloxyphenol Methoxyphenyl Triazin) en une quantité de 0,01 à 10 % en poids, par rapport au poids total de la préparation.

11. Utilisation ou procédé selon l'une quelconque des revendications précédentes, caractérisé ou **caractérisée en ce que** la préparation contient du 4-(tert-butyl)-4'-méthoxydibenzoylméthane (INCI : Butyl Methoxydibenzoylmethane) en une quantité de 0,01 à 10 % en poids, par rapport au poids total de la préparation.

12. Utilisation ou procédé selon l'une quelconque des revendications précédentes, caractérisé ou **caractérisée en ce que** la préparation contient de l'ester hexylique de l'acide (2-[4-(diéthylamino)-2-hydroxybenzoyl]-benzoïque (INCI : Diethylamino Hydroxybenzoyl Hexyl Benzoate) en une quantité de 0,01 à 10 % en poids, par rapport au poids total de la préparation.
